(19) Europäisches Patentamt
European Patent Office
Office européen des brevets

(11) Publication number: **0 037 231**
**B1**

(12) **EUROPEAN PATENT SPECIFICATION**

(45) Date of publication of patent specification: 28.01.87

(51) Int. Cl.[4]: **C 07 D 209/42,**
C 07 D 405/12,
C 07 D 409/12,
C 07 D 401/12, A 61 K 31/40

(21) Application number: 81301243.2

(22) Date of filing: 24.03.81

(54) **Substituted acyl derivatives of octahydro-1H-indole-2-carboxylic acids.**

(30) Priority: 02.04.80 US 137106
06.10.80 US 194307
17.02.81 US 233940

(43) Date of publication of application:
07.10.81 Bulletin 81/40

(45) Publication of the grant of the patent:
28.01.87 Bulletin 87/05

(84) Designated Contracting States:
AT BE CH DE FR GB IT LI LU NL SE

(73) Proprietor: WARNER-LAMBERT COMPANY
201 Tabor Road
Morris Plains New Jersey 07950 (US)

(72) Inventor: Hoefle, Milton Louis
2108 Brockman Boulevard
Ann Arbor Michigan 48104 (US)
Inventor: Bobowski, George
3376 Tacoma Circle
Ann Arbor Michigan 48104 (US)

(74) Representative: Jones, Michael Raymond et al
HASELTINE LAKE & CO. Hazlitt House
28 Southampton Buildings Chancery Lane
London WC2A 1AT (GB)

(56) References cited:
EP-A-0 012 401
EP-A-0 012 845
EP-A-0 018 104
EP-A-0 024 852
EP-A-0 029 488
EP-A-0 031 741
BE-A- 608 905

(56) References cited:
BE-A- 611 886
GB-A-2 042 535
US-A-4 105 776

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 037 231 B1

**Description**

This invention relates to substituted acyl derivatives of octahydro-1H-indole-2-carboxylic acids.

1-(3-Mercapto-2-methyl-propanoyl)-pyrrolidine-2-carboxylic acid (Captopril) is a known ACE inhibitor having an IC50, as determined in vitro by the Cashman's method, of $2 \times 10^{-8}$.

The invention provides octahydro-1-(ω-mercaptoalkanoyl)-1H-indole-2-carboxylic acid compounds having the formula:

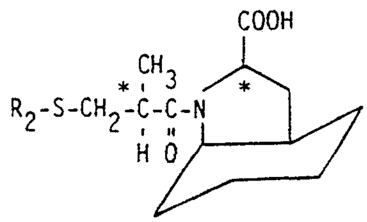

wherein $R_2$ is a hydrogen atom or

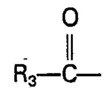

in which $R_3$ is a lower alkyl radical having from 1 to 4 carbon atoms;

a lower, $C_{1-4}$, alkyl ester thereof, or a pharmaceutically acceptable salt thereof.

Preferred compounds of the invention are octahydro-1-(ω-mercaptoalkanoyl)-1H-indole-2-carboxylic acids having the formula:

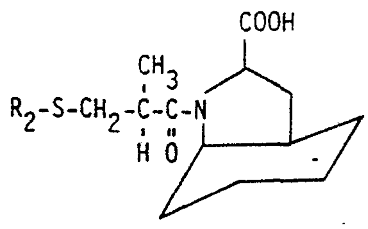

wherein $R_2$ is a hydrogen atom or

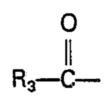

in which $R_3$ is a lower alkyl radical having one or two carbon atoms;

a lower, $C_{1-4}$, alkyl ester thereof, or a pharmaceutically acceptable salt thereof. The preferred specific compounds of the invention are:

octahydro-1-(3-mercapto-2-methylpropanoyl)-1H-indole-2-carboxylic acid;

octahydro-1-[3-(acetylthio)-2-methylpropanoyl]-1H-indole-2-carboxylic acid;

octahydro-1-[3-(propanoylthio)-2-methylpropanoyl]-1H-indole-2-carboxylic acid;

and the pharmaceutically acceptable basic salts thereof.

The compounds of the invention have asymmetric carbon atoms. These carbon atoms are indicated by an asterisk in formula I. Additional asymmetric carbon atoms may be present in the lower alkyl groups. The compounds accordingly exist as optical isomers and diastereomers or as racemates and mixtures thereof. All of these are within the scope of the invention.

Single crystal x-ray diffraction analysis of the N-3-bromobenzoyl derivative of octahydro-1H-indole-2-carboxylic acid used as a starting material in this invention has shown that the cyclohexane and pyrrolidine ring junction is the *cis* configuration, with the carboxylic acid group of the pyrrole ring disposed *cis* to the fused cyclohexane ring, i.e.,

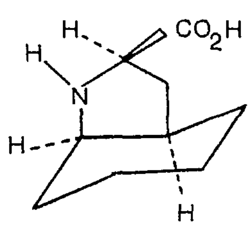

Furthermore, octahydro-1H-indole-2-carboxylic acid has been resolved *via* the α-phenylethylamine salt of its N-benzoyl derivative. Biologically active compounds are derived from either the racemic or levorotatory forms of octahydro-1H-indole-2-carboxylic acid. Optical isomers and diastereomers arising from the chirality at the centers marked with an asterisk in formula I and racemates and mixtures thereof are within the scope of this invention. The *S* configuration at these centers is preferred.

The compounds of the invention may exist in anhydrous form as well as in solvated, including hydrated, forms. In general, the hydrated forms and the solvated forms with pharmaceutically acceptable solvents are equivalent to the anhydrous or unsolvated form for the purposes of the invention.

The compounds of the invention which have the formula

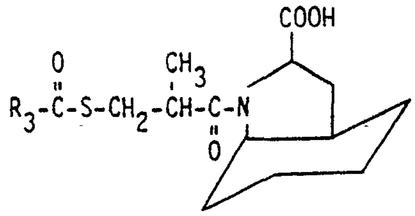

or the lower, $C_{1-4}$, alkyl esters thereof are produced by reacting octahydro-1H-indole-2-carboxylic acid or the lower, $C_{1-4}$, alkyl ester thereof with an

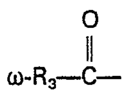

mercaptoalkanoic acid halide of the formula,

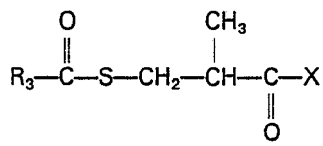 II

in a basic medium; where X is halogen, preferably chlorine or bromine; and $R_3$ has the significance specified above. The basic media can be provided by the use of two molar equivalents of the octahydro-1H-indole-2-carboxylic acid compound or more preferably by the use of an excess of a tertiary organic amine such as pyridine or triethylamine, an alkali or alkaline earth metal hydroxide, an alkali metal bicarbonate, an alkali metal carbonate or other inorganic base capable of neutralizing the hydrogen halide formed during the reaction. The reaction is carried out at a temperature of about 0°C to about 45°C under anhydrous or aqueous conditions. Suitable organic solvents for the reaction include dichloromethane, tetrahydrofuran, dioxane, chloroform, pyridine and triethylamine. The reaction is quite rapid and is usually complete in about one-half to four hours.

The compounds of the invention wherein $R_2$ is an

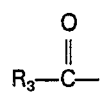

group and R is hydrogen can, in accordance with the invention, also be produced by reacting a trimethylsilyl ester of a octahydro-1H-indole-2-carboxylic acid with an

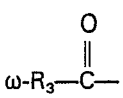

mercaptoalkanoic acid halide (II) followed by hydrolysis of the intermediate trimethylsilyl ester compound to the free acid by treatment with water. The first step of the process is carried out in a non-protic solvent such as methylene chloride, tetrahydrofuran, dioxane, chloroform or acetonitrile at an elevated temperature, usually about 60°C to 100°C. After the reaction is complete, about one-half to one hour, the intermediate trimethylsilyl ester compound is treated with water at room temperature to produce the desired product.

The compounds of the invention which have the formula,

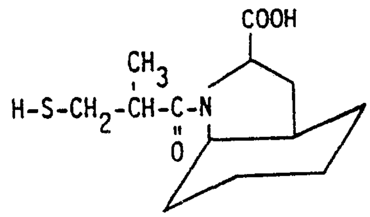

III

and the lower, $C_{1-4}$, alkyl esters thereof can, in accordance with the invention, be produced by hydrolyzing a compound of the invention which has the formula,

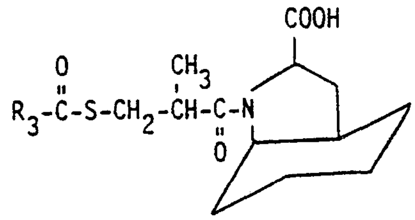

or the lower, $C_{1-4}$, alkyl ester thereof where $R_3$ has the same significance as given above. The hydrolysis is most conveniently carried out by reacting said compound with an excess of an alkali metal hydroxide in an aqueous alcoholic solution under an inert atmosphere for 5 minutes to 24 hours at a temperature of about 20°C to about 80°C. The compounds of formula III can also be produced by ammonolysis of a compound of the formula,

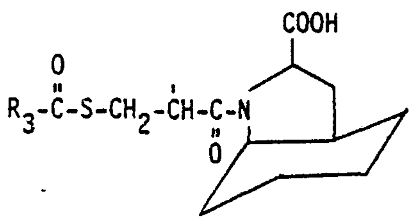

wherein $R_3$ has the same significance as given above. The ammonolysis is most conveniently carried out at room temperature in an alcohol which has been saturated with gaseous ammonia. The reaction usually requires 1 to 24 hours for completion.

The above described synthesis can utilize the racemate or one of the enantiomers as starting material. When the racemic starting material is used in the synthetic procedure, the stereomers obtained in the product can be separated by conventional chromatographic or fractional crystallization methods.

Compounds of the invention of formula I where $R_2$ is

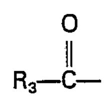

may alternately be prepared from compounds where $R_2$ is hydrogen by treatment of the latter with a suitable acylating agent,

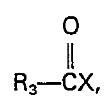

where X is a leaving group; e.g., Cl, Br,

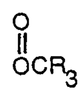 , 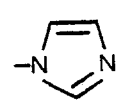 ;

in the presence of a base, e.g., alkali carbonates or tertiary organic amines; in aprotic solvents, e.g., dimethylformamide, tetrahydrofuran or chlorinated hydrocarbons. These may be purified as the free acids or isolated as salts with hindred organic amines, e.g., dicyclohexylamine or t-butylamine.

The products are obtained typically as a mixture of diastereomers which can be separated by standard methods of fractional crystallization or chromatography.

The compounds of this invention form basic salts with various inorganic and organic bases which are also within the scope of the invention. Such salts include ammonium salts, alkali metal salts like sodium and potassium salts, alkaline earth metal salts like the calcium and magnesium salts, salts with organic bases, e.g., dicyclohexylamine or benzathine, salts with basic amino acids like arginine, lysine and the like. The pharmaceutically acceptable salts are preferred, although other salts such as the dicyclohexylamine salt are also useful, e.g., in isolating, purifying or characterizing the product.

The salts are formed in conventional manner by reacting the free acid form of the product with one or more equivalents of the appropriate base providing the desired cation in a solvent or medium in which the salt is insoluble, or in water and removing the water by freeze drying.

In the compounds of formula I when $R_3$ is heteroaryl containing 1 or 2 nitrogen atoms the pharmaceutically acceptable acid addition salts may be prepared by conventional reactions with equivalent amounts of organic or inorganic acids. As exemplary, but not limiting, of pharmaceutically acceptable acid salts are the salts of hydrochloric, sulfuric, acetic, fumeric, malic, maleic and citric acids.

The action of the enzyme renin on angiotensinogen, a pseudoglobulin in blood plasma, produces the decapeptide angiotensin I. Angiotensin I is converted by angiotensin converting enzyme (ACE) to the octapeptide angiotensin II. The latter is an active pressor substance which has been implicated as the causative agent in various forms of hypertension in various mammalian species, e.g., rats and dogs. The compounds of this invention intervene in the renin → angiotensin I → angiotensin II sequence by inhibiting angiotensin I converting enzyme and reducing or eliminating the formation of the pressor substance angiotensin II, and therefore are useful in reducing or relieving hypertension. Thus by the administration of a composition containing one or a combination of compounds of formula I or a pharmaceuctically acceptable salt thereof, hypertension in the species of mammal suffering therefrom is alleviated. A single dose, or preferably two to four divided daily doses, provided on a basis of about 0.1 to 100 mg per kilogram per day, preferably about 1 to 50 mg per kilogram per day is appropriate to reduce blood pressure. The substance is preferably administered orally, but parenteral routes such as subcutaneously, intramuscularly, intravenously or intraperitoneally can also be employed.

The following Table shows the *in vitro* activity of compounds of formula V in an assay for angiotensin converting enzyme inhibitory activity which is a modification of a test reported by D. Cushman and H. Cheung, Biochemical Pharmacology, *20*, 1637—1648 (1971).

*In vitro ACE Assay:* Angiotensin converting enzyme (ACE) inhibitory activity is determined by assaying guinea pig serum ACE in the presence and absence of the test compound. ACE from guinea pig serum and the test compounds are preincumbated for 10 minutes before the addition of the labelled substrate $^3$H-hippuryl-glycyl-glycine. After a 60 minute incubation at 37°C the reaction is stopped by the addition of 0.1N HCl. ACE cleaves the hippuryl-glycyl bond to form the dipeptide glycyl-glycine and $^3$H-hippuric acid. The $^3$H-hippuric acid is then extracted with ethyl acetate and the ACE inhibition of a given sample calculated on the basis of the $^3$H-hippuric acid generated.

TABLE
Activity of Compounds of Formula V

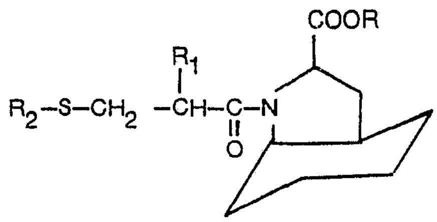

V

| R | $R_1$ | $R_2$ | Diastereoisomer | $IC_{50}$ Molar Conc. |
|---|---|---|---|---|
| $C_2H_5$ | $CH_3$ | $CH_3CO—$ | — | $6.7 \times 10^{-6}$ |
| H | $CH_3$ | H | — | $1.6 \times 10^{-8}$ |
| H | $CH_3$ | $CH_3CO—$ | A | $1.6 \times 10^{-7}$ |
| H | $CH_3$ | $CH_3CO—$ | dl, B | $1.1 \times 10^{-6}$ |
| H | $CH_3$ | H | dl, A | $7.0 \times 10^{-9}$ |
| H | $CH_3$ | $CH_3CH_2CO$ | dl, A | $5.2 \times 10^{-8}$ |
| H | $CH_3$ | $(CH_3)_3CCO$ | dl, A | $1.7 \times 10^{-7}$ |
| H | $CH_3$ | H | l, A | $5.2 \times 10^{-0}$ |

The $IC_{50}$ is the molar concentration of compound which inhibits 50% of the conversion of angiotensin *I* to angiotension *II*.

The compounds of the invention can be utilized to reduce blood pressure in the form of tablets, capsules or elixirs for oral administration or in sterile solutions or suspensions for parenteral administration. About 10 to 500 mg. of a compound or mixture of compounds of formula I or a pharmaceutically acceptable salt thereof is compounded with a pharmaceutically acceptable vehicle or carrier which may contain excipients, binders, preservatives, stabilizers, flavors, etc., in accord with accepted pharmaceutical practice. The amount of active substance in these compositions or preparations is such that a suitable dosage in the range indicated is obtained.

Illustrative of the inert ingredients which may be incorporated in tablets, capsules and the like are the following: A binder such a gum tragacanth, acacia, corn starch or gelatin; an excipient such as dicalcium phosphate; a disintegrating agent such as corn starch, potato starch, alginic acid and the like; a lubricant such as magnesium stearate; a sweetening agent such as sucrose, lactose or saccharin; a flavoring agent such as peppermint, oil of wintergreen or cherry. When the dosage unit form is a capsule, it may contain in addition to materials of the above type a liquid carrier such as a fatty oil. Various other materials may be present as coatings or to otherwise modify the physical form of the dosage unit. For instance, tablets may be coated with shellac, sugar or both. A syrup or elixir may contain the active compound, sucrose as a sweetening agent, methyl and propyl parabens as preservatives, a dye and a flavoring such as cherry or orange flavor.

Sterile compositions for injection can be formulated according to conventional pharmaceutical practice by dissolving or suspending the active substance in a vehicle such as water for injection, a naturally occurring vegetable oil like sesame oil, coconut oil, peanut oil, cottonseed oil, etc., or a synthetic fatty vehicle like ethyl oleate or the like. Buffers, preservatives, antioxidants and the like can be incorporated as required.

The invention is illustrated by the following examples.

## Example 1

Ethyl (2α,3aβ,7aβ)-Octahydro-1-[3-(acetylthio)propanoyl]-1H-indole-2-carboxylate

A solution of 4.2 g (0.025 mole) of 3-(acetylthio)propanoyl chloride in 10 ml of dichloromethane is added over a period of 20 minutes to a stirred mixture of 4.9 g (0.025 mole) of racemic ethyl (2α,3aβ,7aβ)-octahydro-1H-indole-2-carboxylate and 4.0 g of sodium bicarbonate in 40 ml of water and 90 ml of dichloromethane at 5 to 10°C with cooling. After 40 minutes the two phases are separated and the aqueous phase extracted with 50 ml of dichloromethane. The combined organic extracts are washed first with dilute

sulfuric acid (0.01N), then sodium bicarbonate (0.01N), and finally water. The organic layer is separated and dried over sodium sulfate. The solvent is removed *in vacuo* giving 5.8 g of product as a viscous colorless liquid, b.p. 245—247°C (753 mm). Infrared (film): 1745 (ester C=O), 1690 (S-acetyl C=O), 1643 $cm^{-1}$ (amide C=O). Tlc (2:1 acetonitrile-methanol/$SiO_2$) single spot, $R_f$ 0.3.

| | | | |
|---|---|---|---|
| Anal. Calcd. for $C_{16}H_{25}NO_4S$: | C, 58.70; | H, 7.70; | N, 4.28. |
| Found: | C, 58.17; | H, 7.69; | N, 4.00. |

The ethyl (2α,3aβ,7aβ)-octahydro-1H-indole-2-carboxylate used as the starting material in Example 1 is prepared by the following procedure. A solution of 100 g (0.53 mole) of ethyl indole-2-carboxylate in 1000 ml of absolute ethanol and 32 ml of concentrated sulfuric acid is hydrogenated over rhodium on charcoal (4.0 g; 10%) using a Paar hydrogenation apparatus until the uptake of hydrogen ceases (22.3 hours). The catalyst is removed by filtration, and the filtrate is evaporated *in vacuo*. The syrupy residue is dissolved in ice water and the solution is neutralized first with potassium carbonate and then made basic with potassium bicarbonate. The oily precipitate is extracted with 300 ml of diethyl ether, and the aqueous layer is extracted with another 300 ml of diethyl ether. The combined ethereal extracts are washed with saturated aqueous sodium chloride solution, dried over sodium sulfate and evaporated *in vacuo* yielding 78.5 g of ethyl (2α,3aβ,7aβ)-octahydro-1H-indole-2-carboxylate as a nearly colorless oil of high purity. The original aqueous layer is saturated with solid sodium chloride and extracted twice with 150 ml of ethyl acetate. The combined extracts are washed with a small amount of water, dried over sodium sulfate and evaporated *in vacuo* to yield an additional 6.5 g of product. Infrared (film): 3450, 3300 (NH), 1732 $cm^{-1}$ (ester C=O). $n_D^{25}$ = 1.4767. This material is sufficiently pure to be used directly in the example above.

### Example 3

Ethyl (2α,3aβ,7aβ)-Octahydro-1-[3-(acetylthio)-2-methylpropanoyl]-1H-indole-2-carboxylate

Utilizing the procedure described in Example 1, ethyl (2α,3aβ,7aβ)-octahydro-1-[3-(acetylthio)-2-methylpropanoyl]-1H-indole-2-carboxylate is produced from ethyl (2α,3aβ,7aβ)-octahydro-1H-indole-2-carboxylate and 3-(acetylthio)-2-methylpropanoyl chloride. The product is a viscous oil, bp 261.6°C (748 mmHg). Infrared (film), 1742 (ester C=O), 1690 (S-acetyl C=O), 1640 $cm^{-1}$ (amide C=O). Tlc (acetonitrile/$SiO_2$) single spot, $R_f$ 0.6.

| | | | |
|---|---|---|---|
| Anal. Calcd. for $C_{17}H_{27}NO_4S$: | C, 59.81; | H, 7.97; | N, 4.10. |
| Found: | C, 59.55; | H, 7.86; | N, 4.04. |

### Example 4A

(2α,3aβ,7aβ)-Octahydro-1-[3-(acetylthio)-2-methylpropanoyl]-1H-indole-2-carboxylic Acid, Diastereomer A

A solution of 2.05 g (0.01 mole) of (2α,3aβ,7aβ)-octahydro-1H-indole-2-carboxylic acid hydrochloride in 15 ml of anhydrous pyridine is stirred at 0°C, while 1.81 g (0.01 mole) of 3-(acetylthio)-2-methylpropanoyl chloride is added dropwise over a period of 10 minutes. The stirring is continued for 2 hours, and then the solution is adjusted to pH 3.5 by the slow addition of 15 percent sulfuric acid. The resulting precipitate is dissolved in 100 ml of diethyl ether, and the aqueous phase is extracted with an additional 100 ml of ether. The combined ethereal extracts are washed with saturated sodium chloride solution, dried over sodium sulfate, and then concentrated to 15 ml. After standing overnight 1.4 g of white crystals are obtained by filtration; mp 165—7°C. Recrystallization from cyclohexane-ethyl acetate (1:1) gives 0.9 g of highly pure diastereomer A; mp 168.5—170°C. Infrared (KBr): 1742 (COOH), 1689 (S-acetyl C=O), 1648, 1592 $cm^{-1}$ (amide C=O).

| | | | |
|---|---|---|---|
| Anal. Calcd. for $C_{15}H_{23}NOS$: | C, 57.49; | H, 7.40; | N, 4.47. |
| Found: | C, 57.33; | H, 7.15; | N, 4.36. |

### Example 4B

(2α,3aβ,7aβ)-Octahydro-1-[3-(acetylthio)-2-methylpropanoyl]-1H-indole-2-carboxylic Acid, Diastereomer B.

After diastereomer A is obtained by filtration, as set forth in Example 4A, 20 ml of isopropyl ether is added to the original filtrate and the solution is concentrated to 15 ml. Upon cooling 0.7 g of additional product; m- 135—7°C, is obtained. Repeated fractional crystallization alternately from hexane and ethyl acetate gives a pure sample of diastereomer B, as a white solid; mp 151.5—153.5°C.

### Example 5A

(2α,3aβ,7aβ)-Octahydro-1-(3-mercapto-2-methylpropanoyl)-1H-indole-2-carboxylic Acid, Diastereomer A.

(2α,3aβ,7aβ)-Octahydro-1-[3-(acetylthio)-2-methylpropanoyl)-1H-indole-2-carboxylic acid (diastereomer A), 1.0 g, is dissolved at room temperature under nitrogen in 5N ammonia in methanol. This is stirred 2.5 hours and then the solvent is removed at reduced pressure. The residue is taken up in water, acidified with a 10% potassium bisulfate solution, and extracted into ethyl acetate. Drying (magnesium sulfate) and concentration of the organic layer gives diastereomer A of the desired product, which is purified by crystallization from ethyl acetate. The pure A isomer has mp 155—156°C.

### Example 5B

(2α,3aβ,7aβ)-Octahydro-1-(3-mercapto-2-methylpropanoyl)-1H-indole-2-carboxylic Acid, Diastereomer B.

Applying the procedure set forth in Example 4B gives diastereomer B of (2α,3aβ,7aβ)-octahydro-1-(3-mercapto-2-methylpropanoyl)-1H-indole-2-carboxylic acid as a white solid, mp 141—142°C.

## SALTS

*Sodium*

(2α,3aβ,7aβ)-Octahydro-1-(3-mercapto-2-methylpropanoyl)-1H-indole-2-carboxylic acid (5 mg) is dissolved in a solution of water (2.5 ml) and an equivalent amount of 1*N* sodium hydroxide. The solution is freeze dried to obtain the sodium salt.

*Magnesium*

(2α,3aβ,7aβ)-Octahydro-1-(3-mercapto-2-methylpropanoyl)-1H-indole-2-carboxylic acid (5 mg) magnesium oxide (49.5 mg) and water (10 ml) are stirred with slight heating until complete solution is obtained. Then the solvent is removed by freeze drying to obtain the magnesium salt.

*Calcium*

(2α,3aβ,7aβ)-Octahydro-1-(3-mercapto-2-methylpropanoyl)-1H-indole-2-carboxylic acid (5 mg) is dissolved in a mixture of calcium hydroxide (91 mg) and water (10 ml), and the solution is freeze dried to obtain the calcium salt.

*Potassium*

(2α,3aβ,7aβ)-Octahydro-1-(3-mercapto-2-methylpropanoyl)-1H-indole-2-carboxylic acid (5 mg) is dissolved in a mixture of an equivalent amount of potassium bicarbonate and water (10 ml) and freeze dried to obtain the potassium salt.

Example 6

(*l*)-(2α,3aβ,7aβ)-Octahydro-1-(3-mercapto-2-methylpropanoyl)-1H-2-carboxylic Acid (Diastereomer A), Dicyclohexylamine Salt.

Following the procedure of Example 5A, but substituting (*l*)-(2α,3aβ,7aβ)-octahydro-1-(3-(benzoylthio)-2-methylpropanoyl)-1H-2-carboxylic acid (diastereomer A) for (2α,3aβ,7aβ)-octahydro-1-[3-(acetylthio)-2-methylpropanoyl]-1H-indole-2-carboxylic acid, (*l*)-(2α,3aβ,7aβ)-octahydro-1-(3-mercapto-2-methylpropanoyl)-1H-2-carboxylic acid is obtained. This material is purified as the dicyclohexylamine salt; mp 145—148°C, $[\alpha]_D^{23} = -53.5°$ (C = 1, methanol).

Reference Example

(2α,3aβ,7aβ)-Octahydro-1-[3-(2,2-dimethylpropanoylthio)-2-methylpropanoyl]-1H-indole-2-carboxylic Acid.

A mixture of 2.44 g of 2,2-dimethylpropanoic acid, 1,1′-carboyldiimidazole and 50 ml of dry dimethylformamide is prepared and stirred for 1 hour at room temperature until the evolution of gas ceases. A solution of racemic (2α,3aβ,7aβ)-octahydro-1-(3-mercapto-2-methylpropanoyl)-1H-indole-2-carboxylic acid (diastereomer A) and 2.05 g of triethylamine in 20 ml of dimethylformamide is then added and this mixture is stirred for 18 hours at 25°C. The solvent is removed at reduced pressure and the residue is treated with water, acidified with dilute hydrochloric acid and extracted with ethyl acetate. The organic layer is washed with a saturated sodium chloride solution, dried (magnesium sulfate) and concentrated to dryness to give the desired product. This is purified by recrystallization from ethyl acetate and has mp 134—136°C.

Example 7

(2α,3aβ,7aβ)-Octahydro-1-[3-(propanoylthio)-2-methylpropanoyl]-1H-indole-2-carboxylic Acid, (Diastereomer A).

Following the procedure described in the above reference Example, and substituting propionic acid for 2,2-dimethylpropanoic acid, (2α,3aβ,7aβ)-octahydro-1-[3-(propanoylthio)-2-methylpropanoyl]-1H-indole-2-carboxylic acid (diastereomer A) can be obtained. It has mp 170—172°C after recrystallization from acetonitrile.

## Resolution of octahydro-1H-indole-2-carboxylic acid

A solution of 20.0 g of racemic (2α,3aβ,7aβ)-octahydro-1H-indole-2-carboxylic acid in 200 ml of water is cooled in an ice bath and treated dropwise during 1.5 hours simultaneously but separately with 14.4 ml of benzoyl chloride and 120 ml of 2N sodium hydroxide solution, keeping the pH between 6 and 8. The solution is stirred for an additional 30 minutes and the pH is adjusted to 1.8 with 1N hydrochloric acid. Racemic N-benzoyl-(2α,3aβ,7aβ)-octahydro-1H-indole-2-carboxylic acid precipitates and is collected by filtration. Recrystallization from aqueous ethanol gives pure product, mp 191—193°C.

This compound, 87.75 g, is added to a solution of 38.9 g, of (*l*)-α-phenylethylamine in 700 ml of methanol to form a solution. This is diluted with 1250 ml of ethyl acetate and seeded with a crystal of the resolved salt. The mixture begins to precipitate the desired salt. After standing 18 hours at 5°C, the salt, collected by filtration, has mp 212—215°C (dec.) and $[\alpha]_D^{23} = -49.4°$ (C = 1, methanol). Recrystallization from a 2:1 mixture of ethyl acetate and methanol gives product with the same mp and rotation.

The levorotatory salt, 48.2 g, is suspended in a mixture of 884 ml of water and 353 ml of methanol and acidified with dilute hydrochloric acidd to pH 2. After 15 minutes, the initial solid dissolves and a new solid separates. Water, 430 ml, is added and the (*l*)-N-benzoyl-(2α,3aβ,7aβ)-octahydro-1H-indole-2-carboxylic acid is collected by filtration, mp 169—171°C, $[\alpha]_D^{23} = -51.4°$ (C = 1, methanol).

A suspension of the (*l*)-benzoate in 200 ml of 6N hydrochloric acid is heated at reflux for 4 hours. The resulting solution is diluted with 100 ml of water and cooled. Filtration removes precipitated benzoic acid. The filtrate is extracted with chloroform and the pH of the aqueous layer is adjusted to 6.5 with dilute sodium hydroxide solution. Concentration of this to dryness gives a solid which is ground and extracted with anhydrous ethanol. Concentration of the ethanol extract gives (*l*)-(2α,3aβ,7aβ)-octahydro-1H-indole-2-carboxylic acid which may be purified by passing it through an ion exchange resin in the acid form and eluting with 2N ammonium hydroxide, isolating the solid and recrystallizing this from anhydrous ethanol. The pure (*l*)-amino acid has mp 265—266°C (dec.), $[\alpha]_D^{23} = -48.5°$ (C = 1, methanol).

## Example 8

1000 tablets each containing 100 mg of (2α,3aβ,7aβ)-octahydro-1-(3-mercapto-2-methylpropanoyl)-1H-indole-2-carboxylic acid are produced from the following ingredients.

| | |
|---|---|
| (2α,3aβ,7aβ)-octahydro-1-(3-mercapto-2-methylpropanoyl)-1H-indole-2-carboxylic acid | 100 g |
| Corn Starch | 50 g |
| Gelatin | 7.5 g |
| Avicel (microcrystalline cellulose) | 25 g |
| Magnesium Stearate | 2.5 g |

The (2α,3aβ,7aβ)-octahydro-1-(3-mercapto-2-methylpropanoyl)-1H-indole-2-carboxylic acid and corn starch are admixed with an aqueous solution of the gelatin. The mixture is dried and ground to fine powder. The Avicel and then the magnesium stearate are admixed with the granulation. This is then compressed in a tablet press to form 1000 tablets each containing 100 mg of active ingredients.

## Example 9

1000 tablets each containing 200 mg of (2α,3aβ,7aβ)-octahydro-1-(3-mercapto-2-methylpropanoyl)-1H-indole-2-carboxylic acid are produced from the following ingredients.

| | |
|---|---|
| (2α,3aβ,7aβ)-octahydro-1-(3-mercapto-2-methylpropanoyl)-1H-indole-2-carboxylic acid | 200 g |
| Lactose | 100 g |
| Avicel | 150 g |
| Corn Starch | 50 g |
| Magnesium Stearate | 5 g |

The (2α,3aβ,7aβ)-octahydro-1-(3-mercapto-2-methylpropanoyl)-1H-indole-2-carboxylic acid, lactose and Avicel are admixed, then blended with the corn starch. Magnesium stearate is added. The dry mixture is compressed in a tablet press to form 1000, 505 mg tablets each containing 200 mg of active ingredient. The tablets are coated with a solution of Methocel E 15 (methyl cellulose) including as a color a lake containing yellow No. 6.

## Example 10

Two piece No. 1 gelatin capsules each containing 250 mg of (2α,3aβ,7aβ)-octahydro-1-(3-mercapto-2-methylpropanoyl)-1H-indole-2-carboxylic acid are filled with a mixture of the following ingredients.

| | |
|---|---|
| (2α,3aβ,7aβ)-octahydro-1-(3-mercapto-2-methylpropanoyl)-1H-indole-2-carboxylic acid | 250 mg |
| Magnesium Stearate | 7 mg |
| USP Lactose | 193 mg |

Example 11

An injectable solution is produced as follows:

| | |
|---|---|
| (2α,3aβ,7aβ)-octahydro-1-(3-mercapto-2-methylpropanoyl)-1H-indole-2-carboxylic acid, sodium salt | 500 g |
| Methyl Paraben | 5 g |
| Propyl Paraben | 1 g |
| Sodium Chloride | 25 g |
| Water for Injection q.s. | 5 l |

The active substance, preservatives and sodium chloride are dissolved in 3 liters of water for injection and then the volume is brought up to 5 liters. The solution is filtered through a sterile filter and aseptically filled into presterilized vials which are then closed with presterilized rubber closures. Each vial contains 5 ml of solution in a concentration of 100 mg of active ingredient per ml of solution for injection.

**Claims for the Contracting States: BE CH DE FR GB IT LI LU NL SE**

1. An octahydro-1-(ω-mercaptoalkanoyl)-1H-indole-2-carboxylic acid compound having the following general formula:

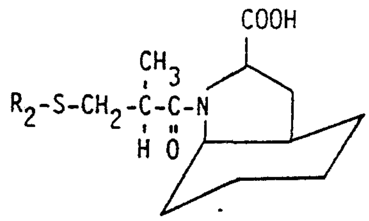

wherein $R_2$ is a hydrogen atom or

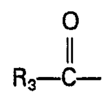

in which $R_3$ is a lower alkyl radical having from 1 to 4 carbon atoms;

a lower, $C_{1-4}$, alkyl ester thereof, or a pharmaceutically acceptable salt thereof.

2. A compound having the following general formula:

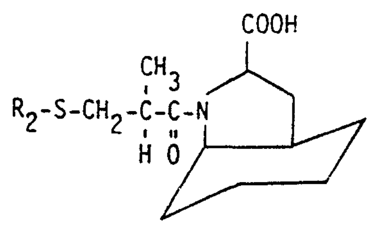

wherein $R_2$ is a hydrogen atom or

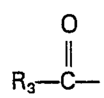

in which $R_3$ is a lower alkyl radical having one or two carbon atoms;

a lower, $C_{1-4}$, alkyl ester thereof, or a pharmaceutically acceptable salt thereof.

3. A compound according to claim 1 or 2, being the compound octahydro-1-[3-mercapto-2-methylpropanoyl]-1H-indole-2-carboxylic acid or a pharmaceutically acceptable basic salt thereof.

4. A compound according to claim 1 or 2, being the compound octahydro-1-[3-(acetylthio)-2-methylpropanoyl]-1H-indole-2-carboxylic acid or a pharmaceutically acceptable basic salt thereof.

5. A compound according to claim 1 or 2, being the compound octahydro-1-[3-(propanoylthio)-2-methylpropanoyl]-1H-indole-2-carboxylic acid or a pharmaceutically acceptable basic salt thereof.

6. A compound according to claim 1, 2 or 3 and being the compound (2α,3aβ,7aβ)-octahydro-1-(3-mercapto-2-methyl-propanoyl)-1H-indole-2-carboxylic acid or a pharmaceutically acceptable basic salt thereof.

7. A compound according to claim 1, 2 or 3, being the compound (2α,3aβ,7aβ)-octahydro-1-(3-mercapto-2-methyl-propanoyl)-1H-indole-2-carboxylic acid, diastereomer A, or a pharmaceutically acceptable basic salt thereof.

8. A compound according to claim 1, 2 or 3 being the compound (*l*)-(2α,3aβ,7aβ)-octahydro-1-(3-mercapto-2-methyl-propanoyl)-1H-indole-2-carboxylic acid, diastereomer A, or a pharmaceutically acceptable basic salt thereof.

9. A process for the production of a substituted octahydro-1-(ω-mercaptoalkanoyl)-1-indole-2-carboxylic acid compound according to claim 1, which comprises acylating an ethyl or trimethylsilyl ester or an acid salt of a tertiary amine or alkali metal of octahydro-1H-indole-2-carboxylic acid with an appropriate acid halide of the formula:

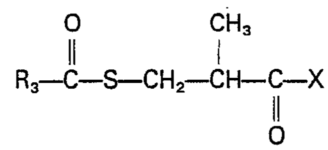

wherein $R_3$ is as defined in claim 1 and X is halogen, and, optionally hydrolyzing with base to obtain a compound according to claim 1 wherein $R_2$ is hydrogen.

10. A pharmaceutical composition comprising a substituted octahydro-1-(3-mercaptoalkanoyl)-1-indole-2-carboxylic acid compound as claimed in any one of Claims 1 to 8, or a pharmaceutically acceptable salt thereof, and a pharmaceutically acceptable carrier.

**Claims for the Contracting State: AT**

1. A process for producing an octahydro-1-(ω-mercaptoalkanoyl)-1-indole-2-carboxylic acid or ester having the formula

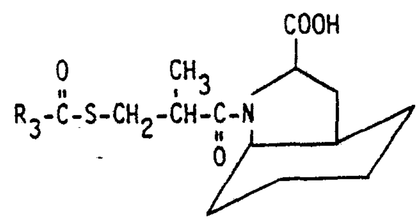

a lower, $C_{1-4}$, alkyl ester thereof or a pharmaceutically acceptable salt thereof wherein $R_3$ is a lower alkyl radical having from 1 to 4 carbon atoms;

which process comprises reacting octahydro-1H-indole-2-carboxylic acid or a lower, $C_{1-4}$, alkyl ester thereof with an acid halide having the formula

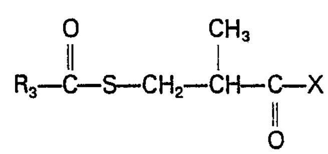

II

wherein X is halogen and $R_3$ is as previously defined, in a basic medium and optionally forming a pharmaceutical salt thereof.

2. A process for producing an octahydro-1-(ω-mercaptoalkanoyl)-1H-indole-2-carboxylic acid having the formula

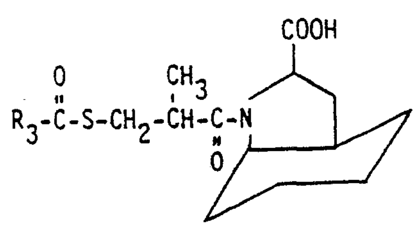

wherein $R_3$ is as defined in claim 1, which process comprises (1) reacting a trialkylsilyl ester of octahydro-1H-indole-2-carboxylic acid with an acid halide having the formula

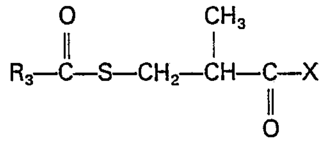

wherein $R_3$ and X are as defined in claim 1, and (2) hydrolysing the intermediate trialkylsilyl ester produced to form the free acid.

3. A process for producing an octahydro-1-(ω-mercaptoalkanoyl)-1H-indole-2-carboxylic acid having the formula

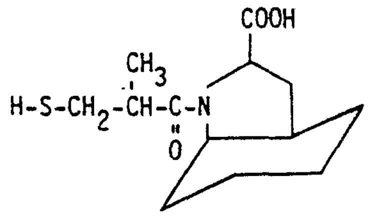

which process comprises hydrolysing an octahydro-1-(ω-mercaptoalkanoyl)-1H-indole-2-carboxylic acid having the formula

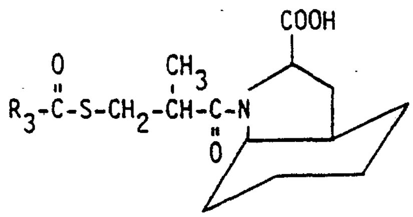

or a lower, $C_{1-4}$, alkyl ester thereof wherein $R_3$ is as defined in claim 1.

4. A process for producing an octahydro-1-(ω-mercaptoalkanoyl)-1H-indole-2-carboxylic acid having the formula

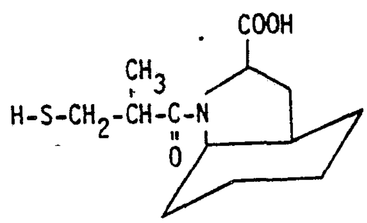

which process comprises subjecting to ammonolysis an octahydro-1-(ω-mercaptoalkanoyl)-1H-indole-2-carboxylic acid having the formula

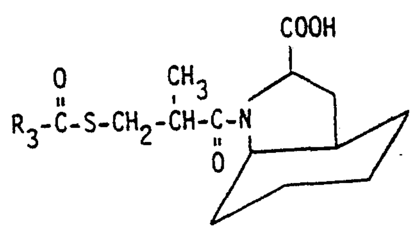

or a lower, $C_{1-4}$, alkyl ester thereof wherein $R_3$ is as defined in claim 1.

5. A process for producing an octahydro-1-(ω-mercaptoalkanoyl)-1H-indole-2-carboxylic acid having the formula

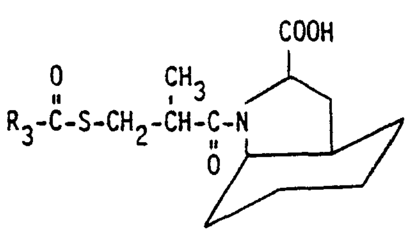

or a lower, $C_{1-4}$, alkyl ester thereof wheein $R_3$ is as defined in claim 1, which process comprises reacting

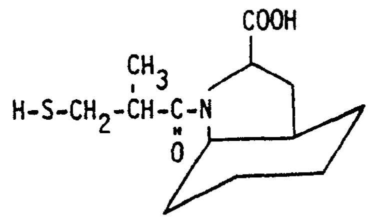

or a lower, $C_{1-4}$, alkyl ester thereof with an acylating agent having the formula

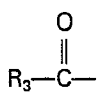

wherein $R_3$ is as defined in claim 1 and X is a leaving group, in the presence of a base.

6. A process for producing an octahydro-1-(ω-mercaptoalkanoyl)-1H-indole-2-carboxylic acid or ester having the formula

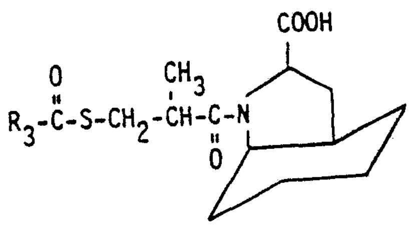

a lower, $C_{1-4}$, alkyl ester thereof or a pharmaceutically acceptable salt thereof wherein $R_3$ is a lower alkyl radical having one or two carbon atoms

which process comprises reacting octahydro-1H-indole-2-carboxylic acid or a lower, $C_{1-4}$, alkyl ester thereof with an acid halide having the formula

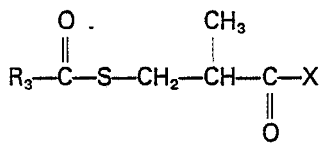

II

wherein X is halogen and $R_3$ is as previously defined, in a basic medium and optionally forming a pharmaceutical salt thereof.

7. A process according to Claim 3 or 4 in which a compound having the following name is prepared: octahydro-1-[3-mercapto-2-methylpropanoyl]-1H-indole-2-carboxylic acid or a pharmaceutically acceptable basic salt thereof.

8. A process according to Claim 3 or 4 in which a compound having the following name is prepared: octahydro-1-[3-(acetylthio)-2-methylpropanoyl]-1H-indole-2-carboxylic acid or a pharmaceutically acceptable basic salt thereof.

9. A process according to Claim 3 or 4 in which a compound having the following name is prepared: octahydro-1-[3-(propanoylthio)-2-methylpropanoyl]-1H-indole-2-carboxylic acid or a pharmaceutically acceptable basic salt thereof.

10. A process according to Claim 3 or 4 in which a compound having the following name is prepared: (2α,3aβ,7aβ)-octahydro-1-(3-mercapto-2-methyl-propanoyl)-1H-indole-2-carboxylic acid or a pharmaceutically acceptable basic salt thereof.

11. A process according to Claim 3 or 4 in which a compound having the following name is prepared: (2α,3aβ,7aβ)-octahydro-1-(3-mercapto-2-methyl-propanoyl)-1H-indole-2-carboxylic acid, diastereomer A, or a pharmaceutically acceptable basic salt thereof.

12. A process according to Claim 3 or 4 in which a compound having the following name is prepared: (*l*)-(2α,3aβ,7aβ)-octahydro-1-(3-mercapto-2-methyl-propanoyl)-1H-indole-2-carboxylic acid, diastereomer A, or a pharmaceutically acceptable basic salt thereof.

13. A process for producing a pharmaceutically acceptable salt of a compound produced by a process according to any of claims 1 to 12, which process comprises reacting the compound with a base or acid capable of forming a pharmaceutically acceptable salt.

**Patentansprüche für die Vertragsstaaten: BE CH DE FR GB IT LI LU NL SE**

1. Octahydro-1-(ω-mercaptoalkanoyl)-1H-indol-2-carbonsäureverbindung mit der folgenden allgemeinen Formel:

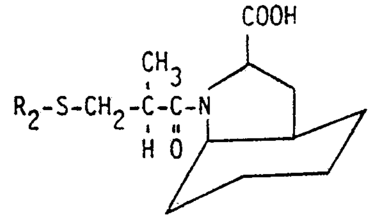

worin $R_2$ ein Wasserstoffatom oder

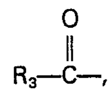

in welchem $R_3$ ein Niedrigalkylradikal mit 1 bis 4 Kohlenstoffatomen darstellt, ist;
ein niederer $C_{1-4}$-Alkylester davon oder ein pharmazeutisch akzeptables Salz davon.

2. Verbindung mit der folgenden allgemeinen Formel:

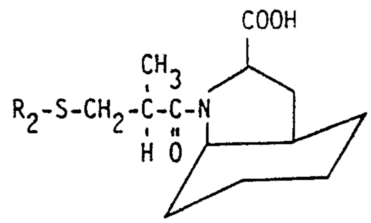

worin $R_2$ für ein Wasserstoffatom oder

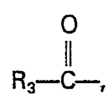

in welchem $R_3$ ein Niedrigalkylradikal mit 1 bis 2 Kohlenstoffatomen darstellt, steht;
ein niederer $C_{1-4}$-Alkylester davon oder ein pharmazeutisch akzeptables Salz davon.

3. Verbindung nach Anspruch 1 oder 2, welche die Verbindung Octahydro-1-[3-mercapto-2-methylpropanoyl]-1H-indol-2-carbonsäure oder ein pharmazeutisch akzeptables Basensalz davon ist.

4. Verbindung nach Anspruch 1 oder 2, welche die Verbindung Octahydro-1-[3-(acetylthio)-2-methylpropanoyl]-1H-indol-2-carbonsäure oder ein pharmazeutisch akzeptables Basensalz davon ist.

5. Verbindung nach Anspruch 1 oder 2, welche die Verbindung Octahydro-1-[3-(propanoylthio)-2-methylpropanoyl]-1H-indol-2-carbonsäure oder ein pharmazeutisch akzeptables Basensalz davon ist.

6. Verbindung nach Anspruch 1, 2 oder 3, welche die Verbindung (2α,3aβ,7aβ)-Octahydro-1-(3-mercapto-2-methyl-propanoyl)-1H-indol-2-carbonsäure oder ein pharmazeutisch akzeptables Basensalz davon ist.

7. Verbindung nach Anspruch 1, 2 oder 3, welche die Verbindung (2α,3aβ,7aβ)-Octahydro-1-(3-mercapto-2-methyl-propanoyl)-1H-indol-2-carbonsäure, Diastereomeres A, oder ein pharmazeutisch akzeptables Basensalz davon ist.

8. Verbindung nach Anspruch 1, 2 oder 3, welche die Verbindung (1)-(2α,3aβ,7aβ)-Octahydro-1-(3-mercapto-2-methyl-propanoyl)-1H-indol-2-carbonsäure, Diastereomeres A, oder ein pharmazeutisch akzeptables Basensalz davon ist.

9. Verfahren zur Herstellung einer substituierten Octahydro-1-(ω-mercaptoalkanoyl)-1-indol-2-carbonsäure-verbindung nach Anspruch 1, welches die Acylierung eines Äthyl- oder Trimethylsilylesters oder eines Säuresalzes eines tertiären Amins oder Alkalimetalls von Octahydro-1H-indol-2-carbonsäure mit einem geeigneten Säurehalogenid der Formel:

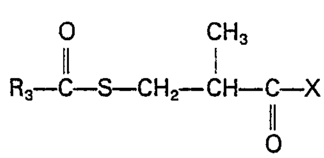

worin $R_3$ wie in Anspruch 1 definiert ist, und X für Halogen steht, und gegebenenfalls die Hydrolyse mit einer Base zum Erhalt einer Verbindung nach Anspruch 1, worin $R_2$ Wasserstoff darstellt, umfaßt.

10. Pharmazeutische Zusammensetzung, welche eine substituierte Octahydro-1-(3-mercaptoalkanoyl)-1-indol-2-carbonsäureverbindung nach irgendeinem der Ansprüche 1 bis 8 oder ein pharmazeutisch akzeptables Salz davon und einen pharmazeutisch akzeptablen Träger umfaßt.

**Patentansprüche für den Vertragsstaat: AT**

1. Verfahren zur Herstellung von Octahydro-1-(ω-mercaptoalkanoyl)-1H-indol-2-carbonsäure oder -ester mit der Formel

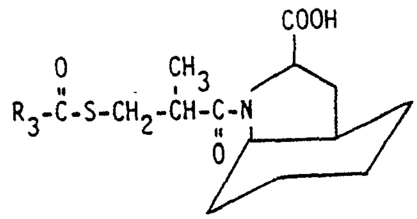

eines niederen $C_{1-4}$-Alkylesters davon oder eines pharmazeutisch akzeptablen Salzes davon, worin $R_3$ ein Niedrigalkylradikal mit 1 bis 4 Kohlenstoffatomen darstellt, welches Verfahren dadurch gekennzeichnet ist, daß es die Umsetzung von Octahydro-1H-indol-2-carbonsäure oder eines niederen $C_{1-4}$-Alkylesters davon mit einem Säurehalogenid der Formel

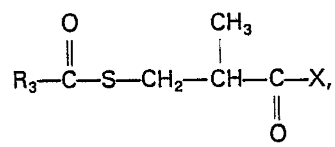

worin X für Halogen steht, und $R_3$ wie vorstehend definiert ist, in einem basischen Medium, und gegebenenfalls die Bildung eines pharmazeutischen akzeptablen Salzes davon umfaßt.

2. Verfahren zur Herstellung einer Octahydro-1-(ω-mercaptoalkanoyl)-1H-indol-2-carbonsäure der Formel

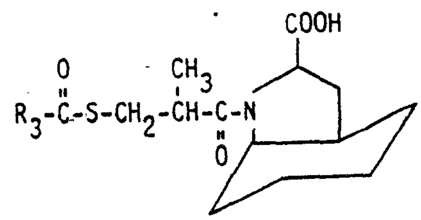

worin $R_3$ die gleiche Bedeutung wie in Anspruch 1 hat, welches Verfahren dadurch gekennzeichnet ist, daß es (1) die Umsetzung eines Trialkylsilylesters von Octahydro-1H-indol-2-carbonsäure mit einem Säurehalogenid der Formel

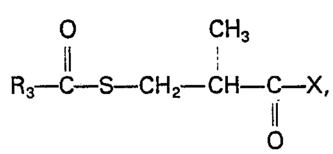

worin $R_3$ und X die gleiche Bedeutung wie in Anspruch 1 haben, und (2) Hydrolysieren des erhaltenen Zwischenprodukts Trialkylsilylester zur Bildung der freien Säure umfaßt.

3. Verfahren zur Herstellung einer Octahydro-1-(ω-mercaptoalkanoyl)-1H-indol-2-carbonsäure mit der Formel

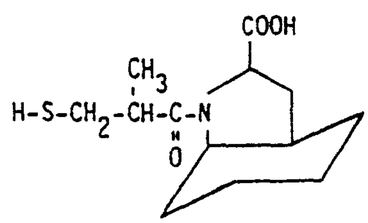

welches Verfahren dadurch gekennzeichnet ist, daß es Hydrolysieren einer Octahydro-1-(ω-mercapto-alkanoyl)-1H-indol-2-carbonsäure der Formel

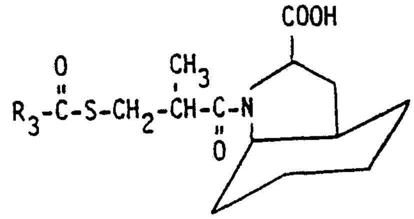

oder eines niederen $C_{1-4}$-Alkylesters davon, worin $R_3$ wie in Anspruch 1 definiert ist, umfaßt.

4. Verfahren zur Herstellung einer Octahydro-1-(ω-mercaptoalkanoyl)-1H-indol-2-carbonsäure der Formel

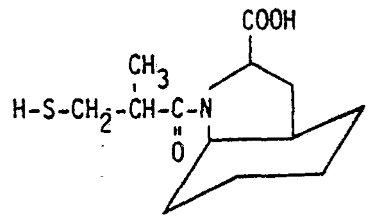

welches Verfahren dadurch gekennzeichnet ist, daß eine Octahydro-1-(ω-mercaptoalkanoyl)-1H-indol-2-carbonsäure der Formel

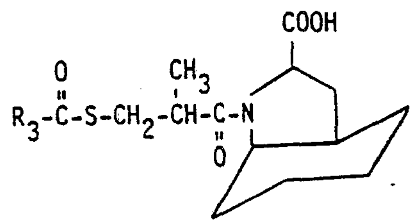

oder eines niederen $C_{1-4}$-Alkylesters davon, worin $R_3$ wie in Anspruch 1 definiert ist, der Ammonolyse unterworfen wird.

5. Verfahren zur Herstellung einer Octahydro-1-(ω-mercaptoalkanoyl)-1H-indol-2-carbonsäure der Formel

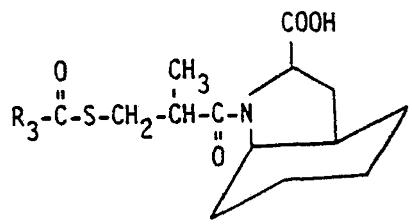

oder eines niederen $C_{1-4}$-Alkylesters davon, worin $R_3$ wie in Anspruch 1 definiert ist, welches Verfahren dadurch gekennzeichnet ist, daß es die Umsetzung von

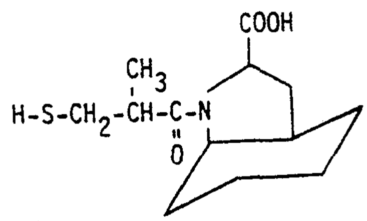

oder eines niederen $C_{1-4}$-Alkylesters davon mit einem Acylierungsmittel der Formel

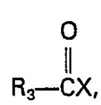

worin $R_3$ wie in Anspruch 1 definiert ist, und X eine austretende Gruppe darstellt, in Gegenwart einer Base umfaßt.

6. Verfahren zur Herstellung von Octahydro-1-(ω-mercaptoalkanoyl)-1H-indol-2-carbonsäure oder -ester der Formel

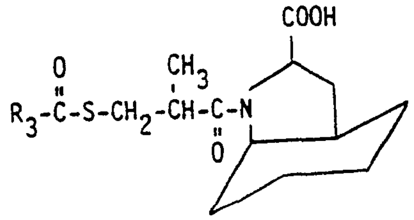

eines niederen $C_{1-4}$-Alkylesters davon oder eines pharmazeutisch akzeptablen Salzes davon, worin $R_3$ ein Niedrigalkylradikal mit 1 oder 2 Kohlenstoffatomen ist, welches Verfahren die Reaktion von Octahydro-1H-indol-2-carbonsäure oder eines niederen $C_{1-4}$-Alkylesters davon mit einem Säurehalogenid der Formel

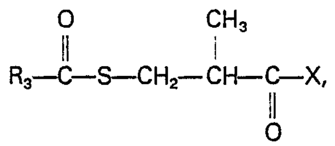

worin X für Halogen steht, und $R_3$ die gleiche Bedeutung wie oben hat, in einem basischen Medium, und gegebenenfalls die Bildung eines pharmazeutisch akzeptablen Salzes davon umfaßt.

7. Verfahren nach Anspruch 3 oder 4, wobei eine Verbindung mit der folgenden Bezeichnung: Octahydro-1-[3-mercapto-2-methylpropanoyl]-1H-indol-2-carbonsäure oder ein pharmazeutisch akzeptables Basensalz davon hergestellt wird.

8. Verfahren nach irgendeinem der Ansprüche 1, 2, 5 oder 6, wobei eine Verbindung mit der folgenden Bezeichnung: Octahydro-1-[3-(acetylthio)-2-methylpropanoyl]-1H-indol-2-carbonsäure oder ein pharmazeutisch akzeptables Basensalz davon hergestellt wird.

9. Verfahren nach irgendeinem der Ansprüche 1, 2, 5 oder 6, wobei eine Verbindung mit der folgenden Bezeichnung: Octahydro-1-[3-(propanoylthio)-2-methylpropanoyl]-1H-indol-2-carbonsäure oder ein pharmazeutisch akzeptables Basensalz davon hergestellt wird.

10. Verfahren nach Anspruch 3 oder 4, wobei eine Verbindung mit der folgenden Bezeichnung: (2α,3aβ,7aβ)-Octahydro-1-(3-mercapto-2-methyl-propanoyl)-1H-indol-2-carbonsäure oder ein pharmazeutisch akzeptables Basensalz davon hergestellt wird.

11. Verfahren nach Anspruch 3 oder 4, wobei eine Verbindung mit der folgenden Bezeichnung: (2α,3aβ,7aβ)-Octahydro-1-(3-mercapto-2-methyl-propanoyl)-1H-indol-2-carbonsäure, Diastereomeres A, oder ein pharmazeutisch akzeptables Basensalz davon hergestellt wird.

12. Verfahren nach Anspruch 3 oder 4, wobei eine Verbindung mit der folgenden Bezeichnung: (1)-(2α,3aβ,7aβ)-Octahydro-1-(3-mercapto-2-methyl-propanoyl)-1H-indol-2-carbonsäure, Diastereomeres A, oder ein pharmazeutisch akzeptables Basensalz davon hergestellt wird.

13. Verfahren zur Herstellung eines pharmazeutisch akzeptablen Salzes einer mittels eines Verfahrens nach irgendeinem der Ansprüche 1 bis 12 hergestellten Verbindung, welches Verfahren die Umsetzung der Verbindung mit einer zur Bildung eines pharmazeutisch akzeptablen Salzes fähigen Base oder Säure umfaßt.

**Revendications pour les Etats contractants: BE CH DE FR GB IT LI LU NL SE**

1. Acide octahydro-1-(ω-mercaptoalcanoyl)-1H-indole-2-carboxylique de formule générale suivante:

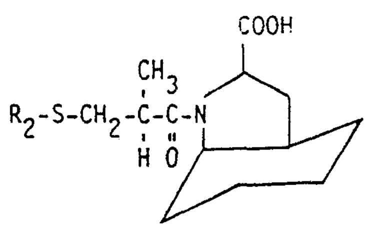

dans laquelle:

$R_2$ représente un atome d'hydrogène ou $R_3$—C(O)— dans lequel $R_3$ représente un radical alkyle inférieur possédant de 1 à 4 atomes de carbone;

un de ses esters d'alkyles inférieurs en $C_1$ à $C_4$ ou un de ses sels pharmaceutiquement acceptables.

2. Un composé de formule générale suivante:

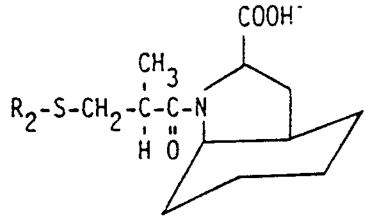

dans laquelle:

$R_2$ représente un atome d'hydrogène ou $R_3$—C(O)— dans lequel $R_3$ représente un radical alkyle inférieur possédant 1 ou 2 atomes de carbone;

un de ses esters d'alkyles inférieurs en $C_1$ à $C_4$ ou un de ses sels pharmaceutiquement acceptables.

3. Un composé selon la revendication 1 ou 2, le composé étant l'acide octahydro-1-[3-mercapto-2-méthylpropanoyl]-1H-indole-2-carboxylique ou un de sels basiques pharmaceutiquement acceptables.

4. Un composé selon la revendication 1 ou 2, le composé étant l'acide octahydro-1-[3-(acétylthio)-2-méthylpropanoyl]-1H-indole-2-carboxylique ou un de sels basiques pharmaceutiquement acceptables.

5. Un composé selon la revendication 1 ou 2, le composé étant l'acide octahydro-1-[3-(propanoylthio)-2-méthylpropanoyl]-1H-indole-2-carboxylique ou un de sels basiques pharmaceutiquement acceptables.

6. Un composé selon la revendication 1 ou 2, le composé étant l'acide (2α,3aβ,7aβ)-octahydro-1-(3-mercapto-2-méthyl-propanoyl)-1H-indole-2-carboxylique ou un de sels basiques pharmaceutiquement acceptables.

7. Un composé selon la revendication 1 ou 2, le composé étant l'acide (2α,3aβ,7aβ)-octahydro-1-(3-mercapto-2-méthyl-propanoyl)-1H-indole-2-carboxylique, diastéréo-isomère A, ou un de sels basiques pharmaceutiquement acceptables.

8. Un composé selon la revendication 1 ou 2, le composé étant l'acide (1)-(2α,3aβ,7aβ)-octahydro-1-(3-mercapto-2-méthyl-propanoyl)-1H-indole-2-carboxylique, diastéréo-isomère A, ou un de sels basiques pharmaceutiquement acceptables.

9. Procédé de préparation d'un acide octahydro-1-(ω-mercaptoalcanoyl)-1-indole-2-carboxylique selon la revendication 1, qui consiste à acyler l'ester d'éthyle ou de triméthylsilyle ou le sel d'amine tertiaire ou d'un métal alcalin de l'acide octahydro-1H-indole-2-carboxylique avec un halogénure d'acide approprié de formule:

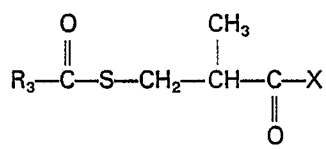

dans laquelle:

$R_3$ est tel que défini dans la revendication 1; et

X représente un atome d'hydrogène,

et éventuellement à hydrolyser avec une base pour obtenir un composé selon la revendication 1 dans lequel $R_2$ représente un atome d'hydrogène.

10. Composition pharmaceutique comprenant un acide octahydro-1-(3-mercaptoalcanoyl)-1-indole-2-carboxylique substitué selon l'une quelconque des revendications 1 à 8, ou un de ses sels pharmaceutiquement acceptables, et un véhicule pharmaceutiquement acceptable.

**Revendications pour l'Etat contractant: AT**

1. Procédé de préparation d'un acide octahydro-1-(ω-mercaptoalcanoyl)-1H-indole-2-carboxylique ou d'un ester de formule:

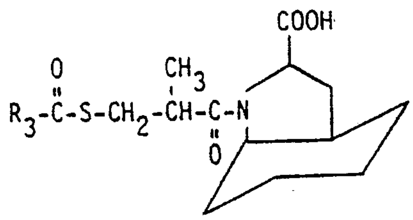

d'un de ses esters d'alkyles inférieurs en $C_1$ à $C_4$ ou d'un de ses sels pharmaceutiquement acceptables, dans lequel $R_3$ représente un radical alkyle inférieur possédant de 1 à 4 atomes de carbone, ce procédé comprenant la réaction d'un acide octahydro-1H-indole-2-carboxylique ou un de ses esters d'alkyles inférieurs en $C_1$ à $C_4$ avec un halogénure d'acide de formule:

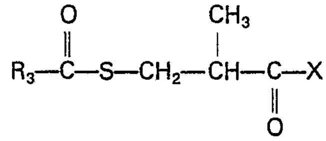

dans laquelle:

X représente un halogène; et

$R_3$ est tel que défini ci-dessus,

en milieu basique et éventuellement à en former un sel pharmaceutiquement acceptable.

2. Procédé de préparation d'un acide octahydro-1-(ω-mercaptoalcanoyl)-1H-indole-2-carboxylique de formule:

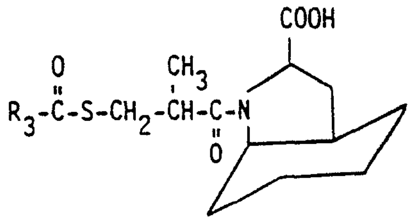

dans laquelle $R_3$ est tel que défini dans la revendication 1, ce procédé consistant:

(1) à faire réagir l'ester de trialkylsilyle de l'acide octahydro-1H-indole-2-carboxylique avec un halogénure d'acide ayant la formule:

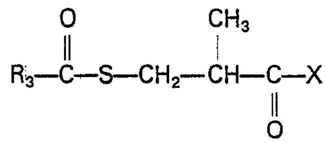

dans laquelle:

$R_3$ et X sont tels que définis dans la revendication 1; et

(2) à hydrolyser l'ester de trialkylsilyle intermédiaire produit pour former l'acide libre.

3. Procédé de préparation d'un acide octahydro-1-(ω-mercaptoalcanoyl)-1H-indole-2-carboxylique présentant la formule:

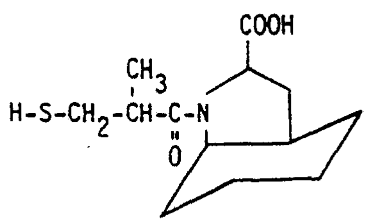

ce procédé consistant à hydrolyser un acide octahydro-1-(ω-mercaptoalcanoyl)-1H-indole-2-carboxylique de formule:

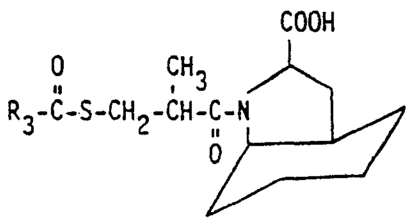

ou un de ses esters d'alkyles inférieurs en $C_1$ à $C_4$, dans lequel $R_3$ est tel que défini dans la revendication 1.

4. Procédé de préparation d'un acide octahydro-1-(ω-mercaptoalcanoyl)-1H-indole-2-carboxylique de formule:

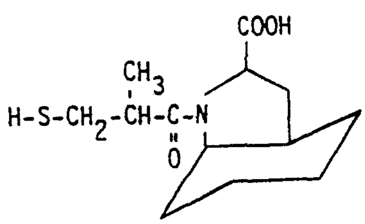

ce procédé consistant à soumettre à une ammonolyse un acide octahydro-1-(ω-mercaptoalcanoyl)-1H-indole-2-carboxylique de formule:

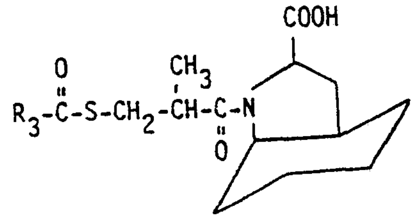

ou un de ses esters d'alkyles inférieurs en $C_1$ à $C_4$, dans lequel $R_3$ est tel que défini dans la revendication 1.

5. Procédé de préparation d'un acide octahydro-1-(ω-mercaptoalcanoyl)-1H-indole-2-carboxylique de formule:

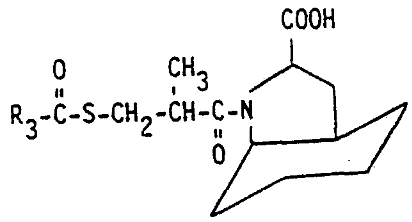

ou d'un de ses esters d'alkyles inférieurs en $C_1$ à $C_4$, dans lequel $R_3$ est tel que défini dans la revendication 1, ce procédé consistant à faire réagir

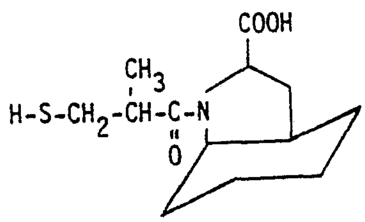

ou un de ses esters d'alkyles inférieurs en $C_1$ à $C_4$ avec un agent d'acylation de formule:

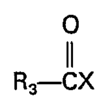

dans laquelle:

$R_3$ est tel que défini dans lâ revendication 1; et

X représente un groupe labile en présence d'une base.

6. Procédé de préparation d'un acide octahydro-1-(ω-mercaptoalcanoyl)-1H-indole-2-carboxylique ou d'un de ses esters de formule:

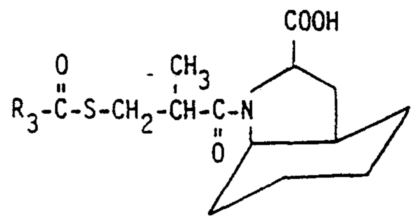

d'un de ses esters d'alkyles inférieurs en $C_1$ à $C_4$ ou d'un de ses sels pharmaceutiquement acceptables, dans lequel $R_3$ est un radical alkyle inférieur possédant 1 ou 2 atomes de carbone, ce procédé consistant à faire réagir l'acide octahydro-1H-indole-2-carboxylique ou un de ses esters d'alkyles inférieurs en $C_1$ à $C_4$, avec un halogénure d'acide de formule:

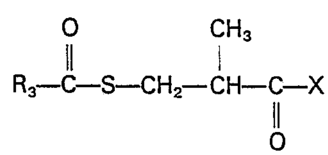

dans laquelle:

X représente un halogène; et

$R_3$ est tel que défini précédemment,

en milieu basique et éventuellement à en former le sel pharmaceutiquement acceptable.

7. Procédé selon la revendication 3 ou 4, dans lequel on prépare un composé de nom suivant: acide octahydro-1-[3-mercapto-2-méthylpropanoyl]-1H-indole-2-carboxylique ou un de ses sels basiques pharmaceutiquement acceptables.

8. Procédé selon l'une quelconque des revendications 1, 2, 5 ou 6, dans lequel on prépare un composé de nom suivant: acide octahydro-1-[3-(acétylthio)-2-méthylpropanoyl]-1H-indole-2-carboxylique ou un de ses sels basiques pharmaceutiquement acceptables.

9. Procédé selon l'une quelconque des revendications 1, 2, 5 ou 6, dans lequel on prépare un composé de nom suivant: acide octahydro-1-[3-(propanoylthio)-2-méthylpropanoyl]-1H-indole-2-carboxylique ou un de ses sels basiques pharmaceutiquement acceptables.

10. Procédé selon la revendication 3 ou 4, dans lequel on prépare un composé de nom suivant: acide (2α,3aβ,7aβ)-octahydro-1-(3-mercapto-2-méthyl-propanoyl)-1H-indole-2-carboxylique ou un de ses sels basiques pharmaceutiquement acceptables.

11. Procédé selon la revendication 3 ou 4, dans lequel on prépare un composé de nom suivant: acide (2α,3aβ,7aβ)-octahydro-1-(3-mercapto-2-méthyl-propanoyl)-1H-indole-2-carboxylique, diastéréo-isomère A, ou un de ses sels basiques pharmaceutiquement acceptables.

12. Procédé selon la revendication 3 ou 4, dans lequel on prépare un composé de nom suivant: acide (1)-(2α,3aβ,7aβ)-octahydro-1-(3-mercapto-2-méthyl-propanoyl)-1H-indole-2-carboxylique, diastéréo-isomère A, ou un de ses sels basiques pharmaceutiquement acceptables.

13. Procédé de préparation d'un sel pharmaceutiquement acceptable d'un composé préparé par un procédé selon l'une quelconque des revendications 1 à 12, ce procédé consistant à faire réagir le composé avec un base ou un acide capable de former un sel pharmaceutiquement acceptable.